## Europäisches Patentamt
## European Patent Office
## Office européen des brevets

(11) Publication number: **0 156 854**
**B1**

# EUROPEAN PATENT SPECIFICATION

(45) Date of publication of patent specification: **05.09.90**

(51) Int. Cl.[5]: **A 61 N 1/32**

(21) Application number: **84903516.7**

(22) Date of filing: **11.09.84**

(86) International application number:
**PCT/US84/01445**

(87) International publication number:
**WO 85/01213 28.03.85 Gazette 85/08**

(54) **NEUROCYBERNETIC PROSTHESIS.**

(30) Priority: **14.09.83 US 531955**

(43) Date of publication of application:
**09.10.85 Bulletin 85/41**

(45) Publication of the grant of the patent:
**05.09.90 Bulletin 90/36**

(84) Designated Contracting States:
**AT BE CH DE FR GB LI LU NL SE**

(73) Proprietor: **ZABARA, Jacob**
**317 Hedgerow Lane**
**Wyncote, PA 19095 (US)**

(72) Inventor: **ZABARA, Jacob**
**317 Hedgerow Lane**
**Wyncote, PA 19095 (US)**

(74) Representative: **Waxweiler, Jean et al**
**OFFICE DENNEMEYER S.à.r.l. P.O. Box 1502**
**L-1015 Luxembourg (LU)**

(56) References cited:
US-A-3 918 461     US-A-37 962 21
US-A-36 502 77     US-A-38 501 61

Journal Thoracic and Cardiovascular Surgery,
vol. 56, no. 1, issued July 1968, Aydin M.
Bilgutay et al, Vagal Tuning; pages 71-82

Surgical Forum, issued 1966, Arnold Neistedt et
al, "Implantable Carotid Sinus Nerve Stimulator
for Reversal of Hypertention", pages 123-127

Annals of Biomedical Engineering, Vol. 8, No.
4-6, issued 1980, Tim Peters et al, "The Principle
of Electrical Carotid ... Therapy", pages 445-458

(56) References cited:
MEDICAL AND BIOLOGICAL ENG. &
COMPUTING, vol. 18, no. 5, September 1980,
pages 660-663; Stevenage, GB S. IORDANESCU
et al.: "Automatic triggering of
neurostimulation as a function of several e.e.g.
parameters."

## Description

### Technical field

The present invention is directed toward a medical prosthesis and more particularly toward a medical prosthesis for alleviating or preventing epileptic seizures and other related clinical conditions of the nervous system such as spasticity.

### Background art

Certain diseases or malfunctions of the nervous system are associated with abnormal neural discharge patterns. Some of these are more or less continuous or chronic, such as is the case with Parkinsonism. Others may be discontinuous, characterized by threshold phenomena, such as in epilepsy. The time of onslaught of grand mal or petit mal seizures is often predictable by neural discharge monitoring or other means, even when the exact causal nature of the precipitating dysfunction is not understood.

It is recognized, however, and confirmed by experimentation, that the introduction of certain control signals of the proper configuration, intensity and duration can act as a means for discharging or modifying hyperactivity in the brain. The superposition of such corrective measures, whether by the generation of proper interference patterns overriding control pulses or cancelling signals, acts in a way to inhibit the normal progress of the seizure and may prevent it altogether. It is also possible that control signals of proper magnitudes when applied to associated neural tracts can cause neural activity to return to its normal state.

Corrective signals of this type can be generated by appropriate electrical pulses or waves applied to neurons. Neurons produce electrochemical signals called action potentials which can be triggered by electronic devices.

The present device can be planted as a neurocybernetic prosthesis in the human for epileptic control. The operation of the prosthesis is based on the principle of augmenting inhibitory processes in the brain to control states of hypersynchronous neural discharge.

Currently, approximately seventy-five percent of epileptics are responsive in some degree to drugs, although undesirable side-effects may force discontinuance. Drug therapy necessitates a continual, general effect on brain cells and other tissues, not infrequently resulting in undesirable side effects whereas epilepsy constitutes an interrupted condition occurring at an approximate average of two convulsions per week. Unlike drug therapy, a neurocybernetic prosthesis can be made operational just during the period of the convulsion by utilizing sensor feedback or manual control. Also, objective evaluation of drug effectiveness involves determination of chemical levels in the blood which is a very costly procedure. Since hyperactivity of the brain is the basis of many nervous system ailments such as Parkinson's disease, cerebral palsy, spasticity, motor disorders, etc., such a prosthesis would also be useful for these chronic nervous illnesses.

An attempt has been made in the past to provide a neurocybernetic prosthesis for alleviating epilepsy and other disorders. It did not, however, meet with much success for several reasons. This prior attempt included a device which had to be implanted into the brain (cerebellum) thereby requiring expensive and extremely risky brain surgery. Furthermore, the implanted device was found to produce tissue trauma in the cranium. It was found that there was a progressive deterioration of cell bodies in the cerebellar cortex due to the electrical current and excessive regeneration of connective tissue.

US—A—3918461 discloses an apparatus for electrically stimulating the human brain by means of electrodes applied directly to the cerebellum in epilepsy treatment.

The object of the present invention is to obviate the need for brain surgery and the resulting tissue trauma caused thereby and to reduce or eliminate an epileptic's dependence on drugs. This object is achieved by means of the neurocybernetic prosthesis claimed in claim 1. Embodiments of the invention are claimed in the dependent claims.

The prosthesis of this invention includes a miniature electronic integrated circuit whose output augments appropriate brain neural discharge to control convulsions or seizures. The circuitry and battery pack are preferably enclosed in an epoxytitanium shell so that the entire device can be totally implanted, preferably in the axilla. Electrode leads pass from the circuit through a subcutaneous tunnel formed toward the neck. The leads terminate in an electrode patch on the vagus nerve.

The device is operated either by sensor feedback or can include manual control and has the capability of regulating aberrant brain signals capable of initiating a convulsive disorder. The principle of design of the neurocybernetic device is based on augmenting central inhibitory processes to regulate the central excitatory state in order to prevent hypersynchronous activity leading to a convulsion. It, therefore, has direct applicability to epilepsy and other nervous system illnesses where convulsions or convulsion-like states obtain.

For the purpose of illustrating the invention, there are shown in the accompanying drawings forms which are presently preferred; it being understood that the invention is not intended to be limited to the precise arrangements and instrumentalities shown.

Figure 1 is a schematic representation of a totally implanted neurocybernetic prosthesis constructed in accordance with the principles of the present invention and showing the manner in which the same is tuned;

Figure 2 is a schematic representation of a partially implanted neurocybernetic prosthesis;

Figure 3 is a schematic representation of a sensor-feedback system for automatically initiating the neurocybernetic prosthesis;

Figure 4 schematically illustrates the placement of an electrode patch on the vagus nerve and the relationship of the vagus nerve with adjacent structures, and

Figure 5 schematically represents the preferred placement of the pulse generator and electrode patch in the human body.

Best mode for carrying out the invention

The present invention operates utilizing a principle called neurocybernetic spectral discrimination and works in the following way. Since, in general, nerves are of a microscopic diameter and are combined together in a nonhomogeneous mixture of diameters and functional properties, it is not presently possible to adequately control external current to selectively activate a specific group of nerves embedded within a relatively large number of other nerves. Spectral discrimination acts to overcome this fundamental problem by "tuning" the external current (electrical generator) to the electrochemical properties of the selected nerves.

The electrochemical properties utilized in the design of the discriminator are: action potential, conduction velocity, refractory period, threshold, resting membrane potential and synaptic transmission. In addition, there are two general properties of the brain called central excitatory state and hypersynchronicity which can be explained in the following manner.

All nerves can be divided into two functional types: excitatory and inhibitory. The spectral discriminator acts to selectively activate those inhibitory nerves which can prevent or block the epileptic seizure. In other words, these specific inhibitory nerves are embedded in a bundle or cable of nerve fibers of varied functions and properties. A bundle of such nerves may typically consist of 100,000 or more individual fibers and contain mixed excitatory and inhibitory characteristics. The purposeful design of the discriminator is to activate just those relatively few nerves which are inhibitory to the epileptic seizure.

Thus, it must be possible to "discriminate" those desired fibers within a broad spectrum of nerves. One reason that this is important is that if excitatory fibers are simultaneously activated with inhibitory fibers then the desired effect of inhibition on the seizure may be nullified. There is a balance of excitation and inhibition in the brain called the central excitatory state which is affected by specific electrochemical signals. Epilepsy is the increase of the central excitatory state to an abnormal level as based on a hypersynchronous discharge of neurons. A second reason for spectral discrimination is to prevent undesirable side-effects by activating other nerves unnecessarily.

There is a physiological basis for the effectiveness of the selected nerves in blocking or preventing epileptic seizures. The activation of these nerves produces an effect on the reticular system via synaptic transmission. The reticular system has been demonstrated to be important in whatever abnormality leads to epileptic seizures. The reticular system is a relatively large and inhomogeneously constituted structure extending from the hind-brain (medulla) to the mid-brain (thalamus) with neural connections to the cerebral cortex and spinal cord. It is not practical at present to directly electrically activate the reticular system because of its large extent and proximity to vital centers. Thus, it was important to discover what nerves might innervate the reticular system sufficiently to produce a significant effect on the reticular system; the net effect being to produce inhibition of epileptic seizures.

For the purpose of interfacing the prosthesis with the critical processes of the brain, inhibition can also be called by its comparable engineering term of negative feedback. Further, it is possible that the seizure originates due to a temporary lack or dimunution of negative feedback from the reticular system to seizure sites in the brain. By acting on appropriately selected nerves, the prosthesis results in the replacement of this negative feedback and thus prevents the seizure.

The approach of spectral discrimination is to utilize the basic properties of conduction velocity, diameter, refractory period, threshold, membrane potential, action potential, after potentials, synchronization and synaptic transmission. Based on thse properties, the parameters of the pulse generator are chosen in terms of frequency, duration of pulse wave, shape of wave, voltage or current and duration of pulse train, In addition, a time-dependent direct current polarization of the membrane can be utilized to produce a "gate" effect.

The "gate" effect is based upon the polarization characteristics of the neural membrane. The membrane potential across the neural membrane can be increased to a point where a block of conduction results. It is a method of separating relatively slower conducting fibers from faster conducting fibers. For example, when the nerve is activated, the action potentials of higher velocity (A) will lead the slower ones (C). A "polarization" block on the nerve membrane will stop A and then the block is removed before C arrives so that the net result is that A, but not C, is prevented from continuing.

The next step is to determine the locus of action of the current generated by the spectral discriminator. This problem relates to the important area of interface between the electronic pulse generator and control signal generated within the brain. In addition, this interface should be of such a nature that the pulse generator is located external to the brain but at the same time the current be set in a compact and identifiable region of nerves so that the site of current is specific and reproducible from patient to patient; no cell bodies are located within the targeted area for current (due to possible production of cell deterioration by the current); and the nerves produce the desired effect on brain operations via sites of synaptic connection.

Analysis by spectral discrimination has demonstrated that the most desirable extra-cranial sites for all these effects are the cranial nerves. Specific cranial serves have been determined to be optimum for beneficial effects on neurological problems. In particular, the vagus nerve is the optimum site for control of epileptic seizures.

If the total spectrum of the nerve is not known, it is possible to activate all the nerve fibers by the spectral discriminator and record the response on an oscilloscope. From this total fiber spectrum, it is possible to determine the settings of the spectral discriminator to select the activation of the appropriate subset of nerves.

Thus, it is possible to identify by the operation of the spectral discriminator those nerves which can produce the desired corrective signal. Spectral discrimination is not only a therapeutic prosthesis method but it is also the method of analysis to determine nervous system sites for beneficial effects in neurological problems.

The present neurocybernetic prosthesis need be turned on only during the duration of a seizure. It can be turned on either manually (by the patient) or automatically by a sensor-feedback system. Many epileptics have sensory signs immediately preceding the convulsion called an aura. At the initiation of the aura, the patient will be able to turn on the device and prevent the seizure. On the other hand, the neurocybernetic prosthesis can include a sensor-feedback system to block the seizure automatically. This feedback system would include sensors specifically designed to determine relatively instantaneous changes in the values of state parameters, which precede eruption of the hypersynchronous activity. Such parameters might include electroencephalographic waves, respiration changes, heart rate changes, various auras or motor effects such as tics or myoclonic jerks. The prosthesis thereby can be activated by sensor feedback producing a signal which precedes convulsive hypersynchronous discharge.

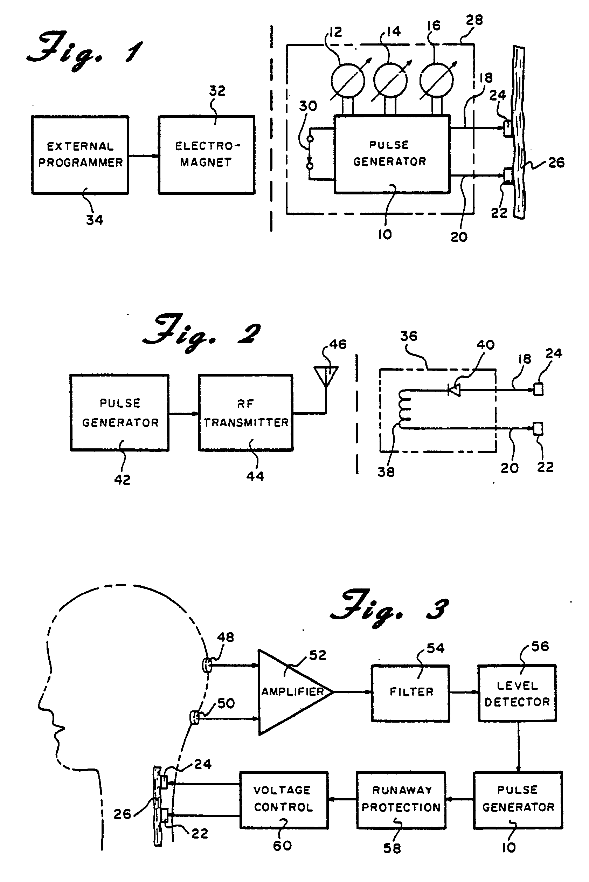

One example of an electrical circuit for practicing the present invention is shown schematically in Figure 1. The circuit is comprised essentially of a pulse generator 10 which is capable of generating electrical pulses having a frequency of between 30 and 80 cyles per second, a pulse duration of between .3 and 1 millisecond and a constant current of between approximately 1 and 10 milliamperes. The frequency, pulse width and the voltage or current level of the output signal from the pulse generator can be varied by controls 12, 14 and 16. Electrode leads 18 and 20 are connected to electrodes 22 and 24 which are applied to the vagus nerve 26 in a manner to be more fully described hereinafter.

In the preferred embodiment of the invention, the pulse generator 10 with its battery pack and other associated circuits are preferably intended to be fully implanted. For this reason, the generator is enclosed in an epoxy-titanium shell 28 (or similar bio-compatible material). As described above, the present invention operates utilizing the principle of neurocybernetic spectral discrimination. The prosthesis must, therefore, combine the desired current parameters to correspond to the specific properties (linear and nonlinear) of the selected nerves. Thus, the command signal of the device is a function of the following specific nerve properties: refractory periods, conduction velocity, synchronization or desynchronization, threshold and brain inhibitory state. In a sense, the current parameters must be "tuned" to the specified nerve properties.

It is for the foregoing reason that the pulse generator 10 is provided with the means 12, 14 and 16 for varying the various current parameters of the pulse signal. The desired parameters are chosen by applying the electrodes 22 and 24 to the vagus nerve and varying the current parameters until the desired clinical effect is produced.

Since this "tuning" may have to be performed after the pulse generator is implanted, the present device includes a means for varying the current parameters percutaneously. This is accomplished by a reed switch 30 associated with the implanted pulse generator 10 which is remotely controlled by electromagnet 32 and external programmer 34. The precise manner in which this is accomplished and the circuitry associated therewith is well known to those skilled in the art as the same technique has been widely used in connection with the "tuning" of cardiac pacemakers.

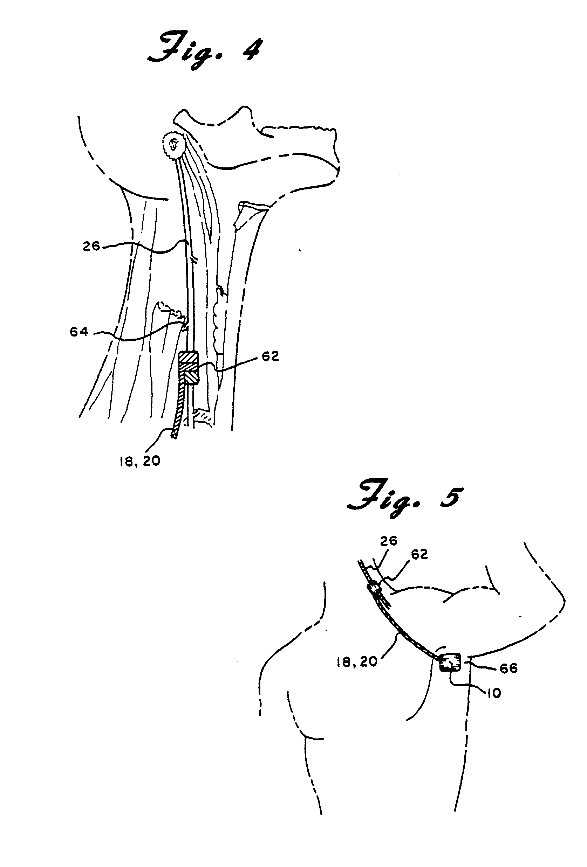

The device shown in Figure 1 is intended for full implantation. It is also possible to practice the present invention with partial implantation. This is accomplished as shown in Figure 2 by the use of a receiver 36 including a coil 38 and diode 40. The receiver is enclosed in an epoxytitanium shell so that it can be implanted and is connected to the electrodes 22 and 24 on the vagus nerve through leads 18 and 20.

Located percutaneously is a pulse generator 42 which modulates the radio frequency transmitter 44 and delivers the radio frequency signal to antenna 46 which transmits the same to the receiver 36 when desired. It should be readily apparent that pulse generator 42 is also capable of being tuned so that the desired current parameters can be obtained. The pulse generator 42, transmitter 44 and antenna 46 could either by permanently worn on a person's body in the vicinity of the receiver 36 so that it need only be turned on when necessary or it may be separately carried in a person's pocket or the like and used whenever needed.

When the neurocybernetic prosthesis of the present invention is utilized for preventing epileptic seizures, it is only necessary for the current generator to be turned on immediately preceding a convulsion. Many epileptics have sensory signs immediately preceding the convulstion called an aura. At the initiation of the aura, the patient will be able to turn on the device to prevent the seizure through the use of a manually operated switch. Even with a fully implanted prosthesis, a momentary contact switch, magnetically operated reed switch or a number of other devices could be provided which could be activated from outside of the body.

It is also possible to provide the prosthesis with a sensor-feedback system to block the seizure automatically. An example of such a system is shown in Figure 3 and includes additional scalp electrodes 48 and 50 for measuring electroencephalographic waves. The output of the electrodes 48 and 50 is amplified by amplifier 52 and is then passed through filter 54 to level detector 56. When level detector 56 senses a significant and predetermined change in the electroencephalographic wave signal, it will automatically initiate the pulse generator 10 which will apply the required pulses to the electrodes 22 and 24 through runaway protection circuit 58 and voltage control circuit 60.

Although the sensing of electroencephalographic waves has been used above as an example for automatically turning on the neurocybernetic prosthesis, it should be apparent that other state parameters can be measured to provide a sensor-feedback system. Such other parameters might include respiration changes, heart rate changes, various auras or motor effects such as tics or myoclonic jerks. As a result, the prosthesis can be activated by sensor feedback producing a signal which precedes convulsive hypersynchronous discharge.

Figure 4 illustrates the placement of the electrodes on the vagus nerve and shows the relationship of the vagus with adjacent structures. The electrodes are shown as a single electrode patch 62 which is known per se. Electrode patch 62 includes both the positive and negative electrodes.

Although it is theoretically possible to place the electrode patch 62 or separate electrodes substantially anywhere along the length of the vagus nerve 26, minimal slowing of the heart rate is achieved by placing the same below the inferior cardiac nerve 64. The electrodes may be placed on or adjacent to the vagus. It is essential, however, that the negative electrode be proximal to the brain and the positive electrode be distal thereto. In certain instances, the positive electrode may be used as an indifferent electrode and be placed in a different part of the body. For example, the case 28 of the implanted pulse generator 10 could, in some instances, be utilized as the positive electrode.

An electrode patch such as that shown in Figure 4 is the preferred embodiment. However, it should be readily apparant to those skilled in the art that various known electrodes could be utilized. The electrodes may be placed either in direct contact with the nerve or in indirect contact with the neural tissue. There is no indication that placement of state of the art electrodes on the nerve itself would have a deleterious effect unless silver electrodes are utilized.

As shown in Figure 5, the axilla or armpit 66 is the preferred location for placement of the pulse generator 10. The axilla provides protection for the pulse generator while allowing freedom of movement and is in proximity to the electrode patch 62. A subcutaneous tunnel between the incision made to implant the electrode patch and the incision made for implanting the pulse generator can be made with a metal rod. A plastic tube can then be inserted in the tunnel through which the electrode leads 18 and 20 can pass without excessive traction.

## Claims

1. A neurocybernetic prosthesis for controlling or preventing involuntary movements such as caused by epileptic seizures, cerebral palsy, Parkinson's disease, spasticity, motor disorders and the like, comprising:

an electrical pulse generator (10) capable of generating pulses having a frequency of between 30 and 80 cycles per second and with each pulse having a duration of between .3 and 1 millsecond;

a single electrode patch (62) including both a positive and negative electrode (22, 24) therein and means (20, 18) electrically connecting said electrode patch (62) to said pulse generator (10), said electrode patch being adapted to be placed in contact with the vagus nerve; and

means including scalp electrodes (48, 50) for sensing electroencephalographic waves and means (54, 56) responsive to a predetermined change in the sensed electroencephalographic waves for turning said pulse generator (10) on.

2. The prosthesis of Claim 1 wherein said pulse generator (10) is enclosed in a shell (28) so that the same can be implanted in a person's body.

3. The prosthesis of Claim 2 further including means for manually turning said generator (10) on when the same is implanted.

4. The prosthesis of Claim 2 or 3 including means (12, 14, 16) for varying the electrical signal generated by said generator (10) from outside a person's body when the generator (10) is implanted.

## Patentansprüche

1. Neurokybernetische Prothese zum Steuern oder Verhindern von unfreiwilligen Bewegungen, wie sie durch epileptische Anfälle, Gehirnlähmung, Parkinson'sche Krankheit, Spastizität, motorische Störungen und dgl. hervorgerufen werden, mit:

einem elektrischen Impulsgenerator (10), der in der Lage ist, Impulse zu erzeugen, die einen Frequenz zwischen 30 und 80 Zyklen pro Sekunde haben und von denen jeder Impuls eine Dauer zwischen 0,3 und 1 Millisekunde hat;

einem einzelnen Elektrodenflecken (62), der sowohl eine positive als auch eine negative Elektrode (22, 24) une eine Einrichtung (20, 18) zum elektrischen Verbinden des Elektrodenfleckens (62) mit dem Impulsgenerator (10) aufweist, wobei der Elektrodenflecken in Kontakt mit dem Vagusnerv plazierbar ist;

eine Einrichtung, die Kopfhautelektroden (48, 50) zum Feststellen von elektroenzephalographischen Wellen und eine Einrichtung (54, 56), welche auf eine vorbestimmte Änderung in den festgestellten elektroenzephalographischen Wellen anspricht, um den Impulsgenerator (10) einzuschalten, aufweist.

2. Prothese nach Anspruch 1, wobei der Impulsgenerator (10) in einen Mantel (28) eingeschlossen ist, so daß er in den Körper einer Person implantiert werden kann.

3. Prothese nach Anspruch 2, weiter mit einer Einrichtung zum manuellen Einschalten des Generators (10), wenn derselbe implantiert ist.

4. Prothese nach Anspruch 2 oder 3, mit einer Einrichtung (12, 14, 16) zum Verändern des elektrischen Signals, das durch den Generator (10) erzeugt wird, von außerhalb eines Körpers einer Person her, wenn der Generator (10) implantiert ist.

## Revendications

1. Prothèse neurocybernétique pour le contrôle ou la prévention de mouvements involontaires dels que ceux qui sont causés par des crises d'épilepsie, une encéphalite, une maladie de Parkinson, une spasticité, des troubles moteurs ou similaires, comprenant:

un générateur d'impulsions électriques (10) capable de produire des impulsions ayant une fréquence comprise entre 30 et 80 Hz, chaque impulsions ayant une durée comprise entre 0,3 et 1 ms;

une pastille d'électrodes (62) contenant à la fois une électrode positive et une électrode négative (22, 24) et des moyens (20, 18) de connexion électrique entre ladite pastille d'électrodes (62) et ledit générateur d'impulsions (10), ladite pastille d'électrodes étant agencée de manière à être placée au contact du nerf pneumogastrique; et

des moyens comprenant des électrodes de cuir chevelu (48, 50) pour détecter des ondes électroencéphalographiques et des moyens (54, 56) qui réagissent à une variation prédéterminée des ondes électroencéphalographiques détectées en mettant en marche ledit générateur d'impulsions (10).

2. Prothèse selon la revendication 1, dans laquelle ledit générateur d'impulsions (10) est enfermé dans une enveloppe (28), de telle sorte qu'il puisse être implanté dans le corps d'une personne.

3. Prothèse selon la revendication 2, comprenant en outre des moyens pour mettre en marche manuellement ledit générateur (10) lorsque celui-ci est implanté.

4. Prothèse selon la revendication 2 ou 3, comprenant des moyens (12, 14, 16) permettant de faire varier de l'extérieur du corps d'une personne le signal produit per ledit générateur (10) lorsque celui-ci est implanté.

*Fig. 1*

EXTERNAL PROGRAMMER — ELECTRO-MAGNET — PULSE GENERATOR

*Fig. 2*

PULSE GENERATOR — RF TRANSMITTER

*Fig. 3*

AMPLIFIER — FILTER — LEVEL DETECTOR

VOLTAGE CONTROL — RUNAWAY PROTECTION — PULSE GENERATOR

Fig. 4

Fig. 5